# EUROPEAN PATENT APPLICATION

(11) **EP 4 253 566 A1**
(43) Date of publication of application: **04.10.2023**
(21) Application number: 22206991.6
(22) Date of filing: 11.11.2022
(51) Int. Cl.: C12Q 1/6883

(54) **MARKER OF CALCIUM-OVERLOAD-MEDIATED NEURONAL DEATH AND APPLICATION THEREOF**

(30) Priority: 02.04.2022 CN 202210351580
(71) Applicant: Capital Medical University, Fengtai District, Beijing 100069 (CN)
(72) Inventor: Liu, Lei, Beijing, 100069 (CN); Gong, Fangyan, Beijing, 100069 (CN)
(74) Representative: London IP Ltd

(57) **Abstract**

The present invention discloses an application of a marker of a calciumoverload-mediated neuronal death in preparation of a drug for treating multiple agingrelated neurodegenerative diseases and in a disease testing method, wherein the diseases include, but are not limited to, an Alzheimer's disease, a Parkinson's disease, amyotrophic lateral sclerosis or glaucoma. The marker of the calcium-overloadmediated neuronal death and the application thereof, used in the present invention, are therapeutically effective for treating the Alzheimer's disease, the Parkinson's disease, the amyotrophic lateral sclerosis and the glaucoma. Moreover, calcium overload and TLK2 hyperphosphorylation may be used as new molecular markers for predicting a disease occurrence progress or testing a therapeutic effect.

## Description

### TECHNICAL FIELD

The present invention relates to the technical field of biomarkers, and, in particular to, a marker of a calcium-overload mediated neuronal death and an application thereof.

### BACKGROUND ART

Alzheimer's disease (AD) and Parkinson's disease (PD) are neurodegenerative diseases most common for the old people. At present, there are five million Alzheimer's disease patients in China, occupying 25% of the total cases in the world; and there are roughly three hundred thousand new cases every year, exhibiting a significant increasing trend. The people over 75 years old have an incidence rate of 8%, and the people over 80 years old have the incidence rate of up to 11%. More female patients suffer from the Alzheimer's disease than male patients, and the number of the female patients over 60 years old, who suffer from the Alzheimer's disease, is 2-3 times higher than that of the male patients. Among the people over 60 years old, the incidence rate of the Parkinson's disease is 1-2%; among the people over 80 years old, the incidence rate of the Parkinson's disease can reach up to 4%, and this disease is a second largest neurodegenerative disease following AD.

Amyotrophic lateral sclerosis is a rare disease, with the incidence rate of hundred thousandth, and about 6,000 new cases appear every year in the world. There are about two hundred thousand patients in China. With regard to the glaucoma, the people over 45 years old have the incidence rate of 2%. Seventy million glaucoma patients have been confirmed in the world, in which there are about ten million glaucoma patients in China.

According to existing researches, calcium overload has a certain function in relevant neurodegenerative diseases, especially in neurons, and calcium ions may control neurotransmitter release, neuronal plasticity and genetic expression, and the like. In the Alzheimer's disease, the intracellular enriching of amyloid-beta proteins may not only directly lead to calcium homeostasis disequilibrium, but also exert an influence on microglial cells and astrocytes in indirect production of the calcium overload. The cellular injury caused by neuronal calcium overload is closely associated with AD occurrence and development. In the amyotrophic lateral sclerosis (ALS), the chronic excitatory toxicity mediated by a calcium-permeability AMPA-type glutamate receptor may cause intracellular calcium-ion unbalance, accompanied by endoplasmic reticulum calcium ion exhaust and mitochondria calcium overload. Moreover, some motor neuron subsets of the brainstem and medulla spinalis only express a small amount of calcium binding proteins, which are especially easily subjected to the calcium-overload injury.

The calcium overload and the change in the expression of channels for controlling calcium homeostasis were found in the lamina cribrosa cells and the trabecular meshwork cells of glaucoma patients. In a rat glaucoma model, the calcium overload was found in the retina ganglian cells.

In the Parkinson's disease, the paradoxical discharge of dopaminergic neurons is maintained by a special Cav1.3 L type calcium channel. The Cav1.3 L type calcium channel needs to be activated by a relatively hyperpolarized potential for calcium ions to enter cells, which accordingly leads to the calcium overload in the dopaminergic neurons and finally to a calcium-dependent neuronal cell death. In the brains of patients suffering from the Parkinson's disease, the abnormal aggregation of alpha-synuclein results in the formation of lewy bodies (Lbs), and the alpha-synuclein aggregation will interact with cytomembranes to form pore channels, resulting in calcium-dependent cell death resulting from calcium dysequilibrium.

Intracellular calcium overload will cause multiple cellular dysfunctions, including cellular oxidation stress increase, nucleus permeability change, hydrolase activity change, ATP level reduction, mitochondrial function damage, DNA incision enzyme activity increase, autophagy function reduction, and the like, and finally result in cell death. In addition, the alpha-synuclein aggregation will also result in the calcium overload and the autophagy function reduction. The autophagy function damage is also an important reason for the occurrence of neurodegenerative diseases such as AD and PD. According to a large number of researches, the genetic factors (e.g., LRRK2, GBA, SMPD1, SNCA, PARK2, PINK1, PARK7 and SCARB2) related to the Parkinson's disease and the genetic factors (e.g., ApoE) related to the Alzheimer's disease all participate in an autophagy-lysosome pathway. These genes participate in relevant functions, for example, lysosomal encoding, lysosome translocation and mitochondria autophagy. The autophagy function damage may directly result in the abnormal aggregation of the alpha-synuclein and beta-the amyloid- protein and the like. Therefore, the maintaining of the cellular autophagy function is also a common target of a neurodegenerative disease in which the calcium overload participates.

### SUMMARY

The present invention aims to provide a marker of a calcium-overload mediated neuronal death and an application thereof (technical effects).

To achieve this objective, the present invention provides an application of a marker of a calcium-overload mediated neuronal death in preparation of a drug for treating multiple aging-related neurodegenerative diseases and in a disease testing method.

Preferably, the diseases include, but are not limited to, Alzheimer's disease, Parkinson's disease, amyotrophic lateral sclerosis or glaucoma.

For the marker of the calcium-overload mediated neuronal death, the marker is hyperphosphorylated TLK2.

Preferably, the marker further includes a TLK2 inhibiting drug, an RNA interference factor of TLK2, a TLK2 knockout factor or a TLK2 knockout RNA interference factor.

Preferably, the TLK2 inhibiting drug includes, but is not limited to, pimozide, thioridazineor trifluoperazine.

Preferably, the RNA interference factor of TLK2 is microRNA interference, antisense RNA or other TLK2 mRNA transcription and protein-translation inhibiting factors.

Preferably, RNA interference sequences of TLK2 are nucleotide sequences as shown in SEQ ID NO. 1-33.

Therefore, the present invention employs the marker of the calcium-overload mediated neuronal death and the application thereof, wherein the calcium overload and TLK2 hyperphosphorylation as new molecular markers for predicting a disease occurrence progress or testing a therapeutic effect not only have a rapid prewarning function for the Alzheimer's disease, the Parkinson's disease, the amyotrophic lateral sclerosis and the glaucoma, but also have a main therapeutic effect in processes of the drugs prepared for treating aforementioned diseases.

In neuronal cells and non-neuronal cells, the calcium overload is induced by an oxygen deficient condition, resulting in TLK2 hyperphosphorylation. In the case of the intracellular calcium overload, hyperphosphorylation occurs at TLK2, which accordingly improves the phosphatase activity thereof. A TLK2 phosphorylation substrate comprises CREBRF (a transcription factor, having a function of controlling lysosome gene transcription) and mitochondria fragmentization caused by an unknown protein. TLK2 gene knockout, RNA interference in TLK2 transcription and protein translation, and a small-molecule inhibitor all may inhibit a downstream injury caused by the calcium overload.

The technical solutions of the present invention will be further described in detail with reference to drawings and embodiments.

### BRIEF DESCRIPTION OF THE DRAWINGS

FIG. 1 is a schematic diagram of Drosophila wing phenotypes;
FIG. 2 a schematic diagram of a fluorescent protein distribution in MC16 Drosophila muscle fiber;
FIG. 3 is a schematic diagram of a calcium-overload phenomenon of MC16 Drosophila;
FIG. 4 is a schematic diagram for TLK RNAi to inhibit a muscle injury caused by calcium overload;
FIG. 5 is a schematic diagram for TLK RNAi to remedy a muscle injury of MC16 Drosophila;
FIG. 6 is a schematic diagram for TLK RNAi to remedy apoptosis caused by calcium overload;
FIG. 7 is a schematic diagram for TLK RNAi to remedy a mitochondrial function defect of an MC16 Drosophila;
FIG. 8 is a schematic diagram of a calcium-overload level in MC16 Drosophila, not influenced by TLK RNAi;
FIG. 9 is a schematic diagram for TLK RNAi to improve an autophagy-lysosome function caused by calcium overload;
FIG. 10 is a schematic diagram for TLK RNAi to remedy dopaminergic neurons caused by alpha-synuclein over-expression;
FIG. 11 is a schematic diagram for TLK RNAi to recover a mitochondrial function in DaGS>SNCA Drosophila;
FIG. 12 is a schematic diagram for TLK RNAi to recover an autophagy-lysosome function caused by alpha-synuclein over-expression;
FIG. 13 is a schematic diagram of autophagy flux activation caused by TLK2 gene knockout;
FIG. 14 is a schematic diagram of an autophagy marker protein caused by TLK2 gene knockout;
FIG. 15 is a schematic diagram for a TLK2 inhibitor (PMZ) to remedy autophagy generated by alpha-synuclein over-expression;
FIG. 16 is a schematic diagram of a chemical structure of a known TLK inhibitor;
FIG. 17 is a schematic diagram for a TLK2 inhibitor (PMZ) to remedy autophagy generated by alpha-synuclein over-expression;
FIG. 18 is a schematic diagram of a TLK2 protein treated with a calcium-overload cell lysis buffer;
FIG. 19 is a schematic diagram of hyperphosphorylation of a TLK2 protein treated with a calcium-overload cell lysis buffer;
FIG. 20 is a schematic diagram of TLK2 kinase activity improved by calcium overload;
FIG. 21 is a schematic diagram of a mouse experiment flow process of a calcium overload induction model;
FIG. 22 is a schematic diagram for TLK2 knockout to delay calcium-overload mouse death time;
FIG. 23 is a schematic diagram for TLK2 gene knockout to remedy a dopaminergic neuron death caused by calcium overload;
FIG. 24 is a schematic diagram for TLK2 gene knockout to improve autophagy function reduction caused by calcium overload;
FIG. 25 is a flow diagram for TLK2 gene knockout to remedy a mouse under an alpha-synuclein over-expression model;
FIG. 26 is a schematic diagram, showing that TLK2 gene knockout can remedy an abnormal behavior caused by alpha-synuclein over-expression;
FIG. 27 is a schematic diagram, showing that TLK2 gene knockout can improve autophagy function reduction caused by alpha-synuclein over-expression;
FIG. 28 is a schematic diagram, showing that TLK2 gene knockout can reduce a dopaminergic neuron death caused by alpha-synuclein over-expression;
FIG. 29 is a schematic diagram of TLK2 hyperphosphorylation under pathological conditions of alpha-synuclein over-expression and calcium overload;
FIG. 30 is a schematic diagram of calcium overload in human-derived retina epidermic cells under a low oxygen condition;
FIG. 31 is a schematic diagram of a discovery that a new micro-molecule has calcium-overload injury inhibition activity;
FIG. 32 is a schematic diagram of mouse TLK2 gene knockout having an effect in glaucoma injury antagonism;
FIG. 33 is a schematic diagram of PMZ having an effect in glaucoma injury antagonism;
FIG. 34 is a schematic diagram, showing that multiple human-derived TLK2 RNAi sequences can reduce a TLK2 protein level;
FIG. 35 is a schematic diagram, showing that TLK2 (shRNA) knockdown can reduce an intracellular tau protein level;
FIG. 36 is a schematic diagram, showing that TLK2 (shRNA) knockdown can reduce an intracellular FUS protein level and improve an autophagy level.

### DETAILED DESCRIPTION OF THE EMBODIMENTS

The technical solutions of the present invention will be further explained with reference to drawings and embodiments.

Unless otherwise defined, technical terms or scientific terms used in the present invention should have the ordinary meaning that can be understood by those of ordinary skill in the art to which the present invention pertains.

For those skilled in the art, it would be obvious that the present invention is not limited to the details of the above exemplary embodiments, and the present invention can be implemented in other specific forms without departing from the spirit or basic characteristics of the present invention. Therefore, from any point of view, the embodiments should be regarded to be exemplary and unrestricted. The scope of the present invention is defined by the appended claims rather than the aforementioned description, and therefore it aims at including all changes within the meaning and scope of the equivalent elements of the claims in the present invention, and any figure signs in the claims should not be regarded to limit the claims involved.

In addition, it should be understood that, although the specification is described according to the implementations, not every implementation only contains one independent technical solution; such description of the specification is only for clearness; and those skilled in the art should regard the specification as a whole, and the technical solutions in each embodiment can also be properly combined to form other implementations which can be understood by those skilled in the art. These other implementations are also included in the protection scope of the present invention.

It should also be understood that the above specific embodiments are only used for explaining the present invention, but the protection scope of the present invention is not limited thereto; and any equivalent replacement or change made by any person skilled in the art in accordance with the technical solutions and concepts of the present invention within the technical scope of the present invention should fall within the present invention/the protection scope of the present invention.

The term "comprising" or "containing" and the like, used in the present invention, aim to mean that an element in front of the term covers the elements listed thereafter, but do not exclude the probability of covering other elements. Orientation or position relationships indicated by terms "medial", "lateral", "superior", "inferior", and the like. are orientation or position relationships shown in the drawings, are adopted not to indicate or imply that indicated devices or components must be in specific orientations or structured and operated in specific orientations but only to conveniently and simply describe the present invention and thus should not be understood as limits to the present invention. After an absolute position of a described object is changed, the relative position relationship also may be correspondingly changed. In the present invention, unless otherwise clearly stipulated and defined, the term "attaching" and other terms should be understood in a broad sense, for example, it can be a fixed connection or a detachable connection or an integrated connection; and it can be directly connected or indirectly connected through an intermediate medium, and it can be an internal communication between two elements or the interaction relationship between two elements. For those of ordinary skill in the art, the specific meaning of the above-mentioned terms in the present invention can be understood according to specific circumstances. The term "about" used in the present invention has the meaning known by those skilled in the art, and preferably means that the numerical value modified by the term is in the range of the numerical value ±50 %, ±40 %, ±30 %, ±20 %, ±10 %, ±5 % or ±1 %.

All terms (including technical terms or scientific terms) used in the present invention have the same meaning as understood by those of ordinary skill in the art to which the present invention pertains, unless otherwise specially defined. It should also be understood that the terms as defined in general dictionaries should be understood as the meaning the same as the meaning thereof in relevant technical context, rather than being explained by the meaning in an idealized or extreme form, unless otherwise clearly defined herein.

For the technologies, methods and apparatuses known by those of ordinary skill in the pertaining art, they may not be discussed in detail, but under proper circumstances, these technologies, methods and apparatuses should be regarded as a part of the specification.

The contents, which are disclosed in the works of literature of the prior art and referred to thereby in the specification of the present invention, are hereby incorporated by reference in the present invention in their entireties and thus become a part of the disclosure of the present invention.

In the following content, unless otherwise specially explained, the drugs and instruments used herein are conventional choices in the art. In addition, information on antibodies and chemical reagents involved in the embodiments is listed in Table 1 and Table 2.

**Table 1 Information of Antibodies Used**

| Antibody Name | Company | Article Number | Type |
|---|---|---|---|
| Anti-α-synuclein | BD Biosciences | 610787 | mouse |
| Anti-TH | Millipore | AB152 | rabbit |
| Anti-Ref(2)p | Abcam | ab178440 | rabbit |
| Anti-GABARAP | Cell Signaling Technology | 13733S | rabbit |
| Anti-p62 | Abcam | ab56416 | mouse |
| Anti-LC3A/B | Cell Signaling Technology | 12741S | rabbit |
| Anti-TLK2 | Proteintech | 13979-1-AP | rabbit |
| Anti-FLAG | Proteintech | 20543-1-AP | rabbit |
| Anti-P-S129-α-Syn | Biolegend | 825701 | mouse |
| Anti-TLK1 | Cell Signaling Technology | 4125S | rabbit |
| Normal IgG | Invitrogen | 10500C | rabbit |

**Table 2 Information of Chemical Reagents referred**

| Name | Company | Article Number |
|---|---|---|
| Goat Anti-mouse IgG-HRP | Gene-Protein Link | P03S01S |
| Goat Anti-rabbit IgG-HRP | Gene-Protein Link | P03S02S |
| Fluo4-AM | Invitrogen | F14201 |
| Pluronic^{™} F-127 | Invitrogen | P3000MP |
| TUNEL | Roche | 12156792910 |
| F-actin | Invitrogen | A12379 |
| TMRM | Invitrogen | T668 |
| Secondary antibody: Alexa Fluor 488 Goat anti-rabbit IgG secondary (H+L) | Invitrogen | A11034 |
| Secondary antibody: Alexa Fluor 488 Goat anti-mouse IgG secondary (H+L) | Invitrogen | A11029 |
| Secondary antibody: Alexa Fluor 568 Goat anti-rabbit IgG secondary (H+L) | Invitrogen | A11036 |
| Secondary antibody: Alexa Fluor 568 Goat anti-mouse IgG secondary (H+L) | Invitrogen | A11031 |
| Lysotracker | Invitrogen | L7528 |
| DAPI | Beyotime | C1005 |
| Immobilon Western HRP Substrate | Millipore | WBKLS0500 |
| PVDF membrane | Millipore | ISEQ00010 |
| RNA extract kit | Beyotime | R0027 |
| cDNA Synthesis SuperMix | TransGen | AH301 |
| SYBR^{™} Green Mater Mix | Applied Biosystems | A25742 |
| Trizol | Invitrogen | 15596026 |
| DMEM | Gibco | 11995081 |
| FBS | Gibco | 10099-141c |
| PS | Gibco | 15140-122 |
| Lipo8000 | Beyotime | C0533 |
| Ionomycin | MedChemExpress | HY-13434 |
| Glutathione Sepharose 4B | GE Healthcare | 17-0756-01 |
| GST-tag Protein Purification Kit | Beyotime | P2262 |
| BL21 chemically competent cells | TransGen | CD701 |
| Protein A/G-agarose beads | Thermo Fisher | 20421 |
| Doxycycline | Sigma | D9891 |
| Tamoxifen | Sigma | T5648 |
| PI | Sigma | P4170 |

In the following content, when mean comparison is performed on the data of the present invention, a t test and a variance test are based on the following assumption: samples overall follow a normal distribution; and the samples have homogeneity of variance. An unpaired t test is used for comparing two groups for statistical analysis. For comparison among more than two groups, a one-way ANOVA with Tukey's post hoc test is used for determining significance. A Kaplan-Meier test is used in a Log-rank algorithm for survival analysis. GraphPad Prism software is used for statistical analysis. P < 0.05 is regarded to be statistically significant (* P < 0.05, ** P < 0.01, *** P < 0.001 and ****p < 0.0001).

### Experimental Test 1

### I. Construction of a chronic calcium-overload model in Drosophila

By genetically modifying Drosophila in expressing a mutant resulting from which glycine at the 430th site of human BNCla is mutated into cysteine, the mutational expression will lead to a slow increase of the calcium ion concentration in cytoplasms, and the transgenic Drosophila is referred to as UAS-C16 (which means an expressive G430C mutant, and transgenic Drosophila No. 16).

In the Parkinson's disease process, calcium overload may also be found in muscular tissue. Moreover, the muscular tissue is an excitability tissue as the same as neurons and has basically the same molecular pathway, and the Drosophila has a greatly huge muscle tissue, which is favorable for observing phenotypes for genetic screening. The largest muscle tissue is a flight muscle which controls the flight of Drosophila wings. When the flight muscle has any injury, the wings will have a corresponding phenotype. Therefore, a muscle promoter, i.e., a Myosin heavy chain (Mhc) promoter, is used to mediate the expression of a Gal4 protein (Mhc-Gal4 will link hBNC1 G430C to the downstream of UAS). Gal4 as a transcriptional activator protein may be combined to a DNA sequence of UAS to strongly activate the expression of a downstream gene segment of a UAS sequence.

In the present invention, by constructing a Gal4 expression strain driven by a Drosophila tissue specific promoter and a Drosophila strain with a transgenic segment linked to UAS and hybridizing two Drosophila strains, the Drosophila progenies may express the genes linked to a posterior end of UAS in tissue specific cells. A Drosophila Gal4/UAS system is used to construct a chronic calcium-overload model in Drosophila muscle, the genotype thereof is Mhc-gal4>UAS-C16, and the Drosophila is abbreviated as MC16 in the following paragraph.

### II. MC16 Drosophila phenotypes

All Drosophila used thereby was raised with a standard corn flour for the Drosophila as food in a constant-temperature incubator at 25°C. The incubator had a humidity maintained at 60%, and was set as a day-light alternative mode, namely, 2000-3000 lux during the period from 9:00 to 21:00, and 0 lux during the period from 21:00 to 9:00 next day. When the Drosophila was raised for about 7 days, the phenotype of the Drosophila wings was directly observed.

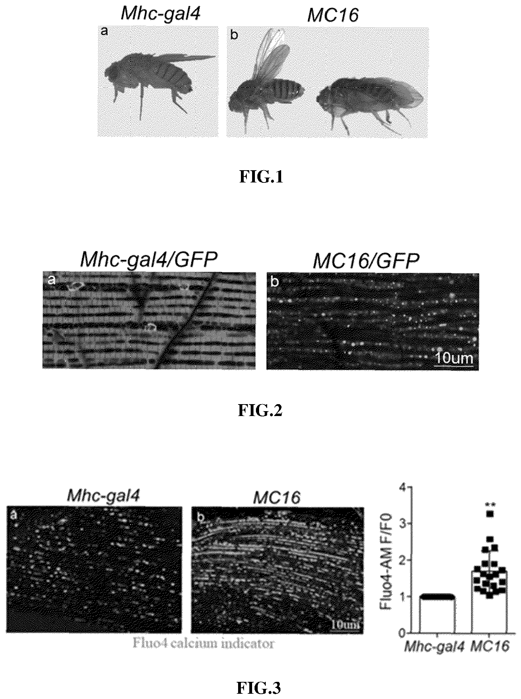

As shown in FIG. 1, normal Mhc-Gal4 Drosophila had the wings parallel to the ground, and could normally fly. MC16 calcium-overload Drosophila continuously drooped downward or held upward the wings in an abnormal muscle state, and lost the flight capability, indicating an injury in the muscle. According to the statistics of the wing phenotypes of MC16 calcium-overload Drosophila on the seventh day, it was found that about 80% of the Drosophila continuously drooped downward or held upward the wings, indicating that MC16 Drosophila could have a corresponding phenotype.

In FIG. 1, FIG. a is Mhc-gal4 Drosophila wings of a normal control in a parallel arrangement, and FIG. b is calcium-overload Drosophila wings at upward or downward positions, which indicates an injury in a wing-controlling muscle.

### III. Muscle fiber of a flight muscle

A Drosophila flight muscle (IFM) was placed and dissected in an HL3 buffer, and since the Drosophila had a green fluorescent protein (GFP), Leica SP8 confocal microscopy could be directly used for imaging.

Since the muscle fiber controlling the flight of the Drosophila wings is at a second thoracic segment, the flight muscle at the thoracic segment was selected to observe muscle fiber morphology. Moreover, the green fluorescent protein (GFP) could be normally uniformly distributed in the cytoplasm.

The Drosophila expressing the green fluorescent protein were respectively hybridized with Mhc-Gal4 Drosophila and MC16 calcium-overload Drosophila, the morphologic change in the muscle fiber tissue could be observed in the Drosophila in the first filial generation. As shown in FIG. 2, FIG. a is Mhc-gal4 Drosophila muscle fiber of a normal control in parallel arrangement, with green fluorescent protein signals uniformly and regularly distributed; in FIG. b, the MC16 Drosophila muscle fiber is disorderly and irregularly distributed, and the green fluorescent protein signals are extremely irregularly distributed, which indicates a muscle injury in the MC16 Drosophila.

### IV. Testing on whether MC16 Drosophila had calcium overload

The Drosophila flight muscle was dissected in the HL3 buffer, and incubated in 5 µM Fluo4-AM and 0.02% Pluronic F-127 in the dark at room temperature for 30 min. Then, the incubated flight muscle was washed with the HL3 buffer for 3 times, and 10 min for each time. The Leica SP8 confocal microscopy was used for imaging and fluorescence intensity measurement.

Fluo4 is a calcium ion indicator, and the fluorescence intensity thereof is enhanced over the increase of calcium ion concentration. Fluo4 was used in the test to verify the calcium ion level. In FIG. 3, F represents a fluorescence intensity, and F/F0 was used for analyzing a relative fluorescence intensity. An unpaired t test was used for two groups, and the difference was statistically significant. The results show that the Fluo4 fluorescence intensity in the MC16 Drosophila was obviously higher than the fluorescence intensity in Mhc-Gal4 Drosophila as the normal control, indicating there was calcium overload in the MC16 Drosophila. Moreover, the calcium-overload signals of the MC16 Drosophila were gathered in mitochondria; because the mitochondria are one of the main organelles for calcium ion metabolism, the calcium overload occurred in the mitochondria.

### V. Capability of TLK RNAi for obviously remedying a phenotype of MC16 Drosophila wings

The UAS-RNAi Drosophila strain was hybridized with the MC16 Drosophila strain, and the Drosophila in the first filial generation was taken. When the Drosophila was raised for about 7 days, the phenotype of the Drosophila wings was directly observed. About 100 pieces of Drosophila were taken for each test, and the test was repeated for three times.

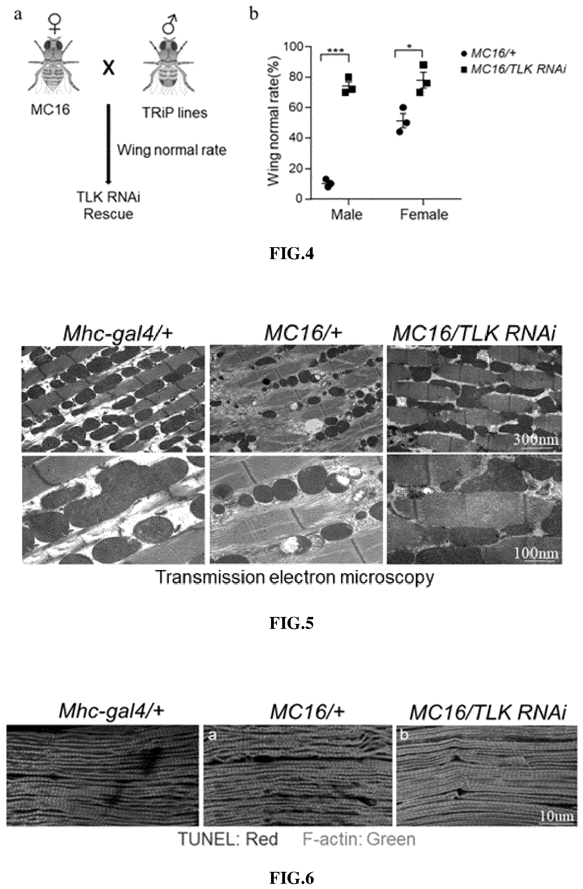

As shown in FIG. 4, FIG. a is a schematic diagram of MC16 Drosophila genetic screening; the MC16 Drosophila was hybridized with the UAS-RNAi Drosophila strains, the phenotype of the MC16 Drosophila wings was observed, and it was found that TLK RNAi could remedy the phenotype of the MC16 Drosophila wings. FIG. b is normal rates of MC16/+ and MC16/TLK RNAi Drosophila in wings, respectively in a comparison between male Drosophila and female Drosophila for 5-7 days. Compared with the MC16/+ Drosophila, the MC16/TLK RNAi Drosophila had a higher wing normal rate.

### VI. Verification of TLK RNAi functions

### (1) TLK RNAi could remedy a muscle injury and a mitochondria defect of the MC16 Drosophila

A Drosophila muscle was fixed in 2.5% glutaraldehyde (diluted by a 0.1 M PB buffer) at 4°C for 4 hours. The muscle was washed in the 0.1 M PB buffer for 3 times, and10 min for each time. Then, samples were prepared for imaging according to standards, and a transmission electron microscopy (HT7700) was used for observation.

As shown in FIG. 5, the transmission electron microscopy was used for observing the muscle fiber and the mitochondria injury of the MC16 calcium-overload Drosophila. The results of the transmission electron microscopy show that the muscle fiber was compact in a long strip and the mitochondria were compact in a round in normal Mhc-Gal4 Drosophila; the muscle fiber was in a dissolved state and the mitochondria were in a vacuolar form in MC16 calcium-overload Drosophila, indicating that the MC16 calcium-overload Drosophila had muscle fiber and mitochondria injuries and that TLK RNAi could remedy the muscle injury and the mitochondria defect of the MC16 Drosophila.

### (2) TLK RNAi could obviously reduce TUNEL staining signals

The Drosophila flight muscle (IFM) was placed and dissected in the HL3 buffer, fixed in 4% PFA at room temperature for 15 min, and then was perforated with 0.2% PBST for three times, with 5 min for each time. Incubation was performed in 10 g/mL proteinase K (diluted by a PBS solution) at 56°C for 10 min. The incubated flight muscle was washed with a 0.2% PBST buffer for 3 times, with 5 min for each time. A Roche TUNEL reaction mixture was prepared according to the specification, and incubation was performed in the dark at 37°C for 2 hours. The incubated flight muscle was washed with a 0.2% PBST buffer for 3 times, with 5 min for each time. F-actin powder was dissolved in DMSO to prepare a 1000-fold stock solution, and the stock solution was diluted in PBS according to 1:1000, stained in the dark at room temperature for 1 hour, and washed with the PBS for three times, with 5 min for each time. The Leica SP8 confocal microscopy was used for acquiring images.

Since the MC16 calcium-overload Drosophila had muscle fiber and mitochondria injuries, these injuries would cause apoptosis . Cell apoptosis and necrosis were respectively tested by terminal deoxynucleotidyl transferase-mediated dUTP nick end labeling (TUNEL) and propidium iodide (PI) staining, so as to determine an apoptosis manner of the MC16 Drosophila. As shown in FIG. 6, the results show that the control Drosophila did not have obvious TUNEL staining signals, the MC16 Drosophila had obvious TUNEL signals, and TLK RNAi could obviously reduce TUNEL staining signals and remedy the apoptosis of the MC16 Drosophila.

### (3) TLK RNAi could recover a mitochondrial function defect caused by calcium overload

The Drosophila flight muscle (IFM) was placed and dissected in the HL3 buffer, stained by 1 µM TMRM and incubated in the dark at room temperature for 10 min. Then, the incubated flight muscle was washed with the HL3 buffer for 3 times, and 10 min for each time.

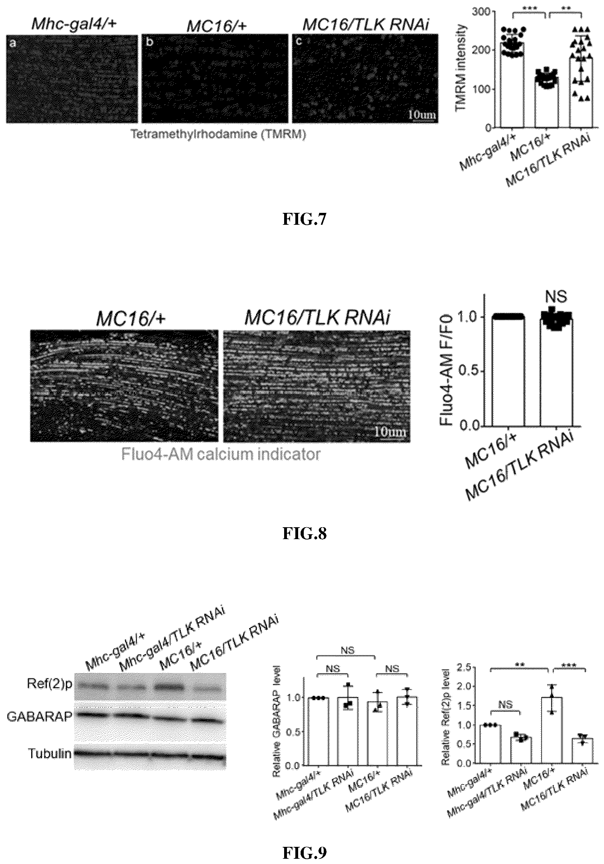

TMRM (Tetramethylrhodamin) is a stain for labeling living cell mitochondria, and is usually used for measuring a mitochondrial membrane potential. In FIG. 7, red represents tetramethylrhodamin (TMRM); fluorescence staining was performed on Mhc-gal4/+, MC16 and MC16/TLK RNAi Drosophila flight muscles by TMRM, and compared with the Mhc-gal4/+ Drosophila, the MC16 Drosophila exhibited a significant reduction in TMRM fluorescence intensity, but TLK RNAi could obviously recover TMRM fluorescence intensity and the mitochondrial function defect caused by the calcium overload.

### (4) TLK RNAi could influence a calcium-overload level

A Drosophila brain or flight muscle was dissected in the HL3 buffer, and incubated in 5 µM Fluo4-AM and 0.02% Pluronic F-127 in the dark at room temperature for 30 min. Then, the incubated brain or flight muscle was washed with the HL3 buffer for 3 times, and 10 min for each time. The Leica SP8 confocal microscopy was used for imaging and fluorescence intensity measurement.

As shown in FIG. 8, by staining with a Fluo4-AM calcium ion indicator, the fluorescence intensity thereof will be enhanced with the increase of calcium ion concentration. The difference in the fluorescence intensity between the MC16/+ Drosophila and the MC16/TLK RNAi Drosophila Fluo4-AM was statistically insignificant, indicating that TLK RNAi did not influence the calcium-overload level.

### (5) TLK RNAi could reduce an elevated Ref(2)P protein level in the MC16 Drosophila

The Drosophila flight muscle was collected, and added in an RIPA lysis buffer added with a protease inhibitor and PMSF (50 mM Tris, 150 mM NaCl, 1% Triton X-100, 1% sodium deoxycholate and 0.1% SDS) for lysis on ice for 30 min. Subsequently, ultrasonication was performed on ice under the conditions of 40 w 3 sec on/3 sec off for 1 minute, centrifugation was performed at 12,000 rpm in a centrifugal machine at 4°C for 15 min, and a supernatant was collected, diluted by an SDS sample-loading buffer and stewed at 95°C for 5 min. 20-30 µg of a protein sample was loaded, and gel electrophoresis was performed under a constant voltage of 80 V. When bromophenol blue reached the bottom, membrane transfer was performed overnight at a constant current of 130 mA to transfer the protein from a gel onto a PVDF membrane. The membrane was blocked with 5% skim milk at room temperature for 1 hour, and washed with TBST for three times, with 5 min for each time. A primary antibody properly diluted was incubated overnight at 4°C, and washed with TBST for three times, with 5 min for each time. An HRP secondary antibody was diluted in 1:10000, incubated at room temperature for 1 hour, and washed with TBST for three times, with 5 min for each time. Western blot was developed by ECL chemiluminescence.

Lysosomes as an intracellular digestive organ could degrade most intracellular substances. Autophagy is a main cytoplasmic protein degradation pathway, and autophagosomes could be fused with the lysosomes to form autolysosomes. When the lysosome function was damaged, an autophagy process would also be also influenced. An LC3 protein complex as an autophagy marker participated in an extension process of an autophagy membrane, and a GABARAP protein having the function similarly to the LC3 protein was necessary for autophagosome maturation.

In addition, Ref(2)P, which is a Drosophila analogue of a mammal animal P62, was used as an autophagy substrate to transfer an ubiquitinated protein to the autophagosome. P62 accumulation usually represents autophagy activity reduction. The Mhc-gal4/+ and the Mhc-gal4/TLK RNAi Drosophila had no significant difference in the protein level. As shown in FIG. 9, compared with the Mhc-gal4/+ Drosophila, the MC16/+ Drosophila had an increased Ref(2)P protein level, and a GABARAP level was not significantly changed, indicating a normal autophagosome process but a probably reduced autolysosome function. Importantly, TLK RNAi could reduce the elevated Ref(2)P protein level in the MC16 Drosophila.

In summary, TLK RNAi could remedy the apoptosis, the mitochondria injury and the autophagy-lysosome reduction induced by the calcium overload, thereby becoming a key molecule for the calcium-overload-mediated cell injury.

### Experimental Test 2

### I. Construction of α-synuclein over-expression Drosophila

A Gaughterless-Gal4 (DaGS) promoter induced UAS-SNCA expression, and the Drosophila involved was abbreviated as DaGS>SNCA. DaGS>SNCA Drosophila could be driven by Mifepristone (RU486) to generally express α-synuclein in the whole body. Since RU486 did not influence the health of the Drosophila, the DaGS>SNCA Drosophila raised with RU486 was compared with the Drosophila not raised with the same. Similarly, the calcium-overload level was tested by Fluo4-AM staining, and the results verified that alpha-synuclein over-expression increased an intracellular calcium ion level of the brain and the muscle of the Drosophila, and the alpha-synuclein over-expression could lead to the calcium overload.

### II. TLK was a key protein for alpha-synuclein aggregation injury

The Drosophila flight systemic tissue was collected, and added in an RIPA lysis buffer added with a protease inhibitor and PMSF (50 mM Tris, 150 mM NaCl, 1% Triton X-100, 1% sodium deoxycholate and 0.1% SDS) for lysis on ice for 30 min. Subsequently, ultrasonication was performed on ice under the conditions of 40 w 3 sec on/3 sec off for 1 minute, centrifugation was performed at 12,000 rpm in a centrifugal machine at 4°C for 15 min, and a supernatant was collected, diluted by an SDS sample-loading buffer and stewed at 95°C for 5 min. 20-30 µg of a protein sample was loaded, and gel electrophoresis was performed under a constant voltage of 80 V. When bromophenol blue reached the bottom, membrane transfer was performed overnight at a constant current of 130 mA to transfer the protein from a gel onto a PVDF membrane. The membrane was blocked with 5% skim milk at room temperature for 1 hour, and washed with TBST for three times, with 5 min for each time. A primary antibody properly diluted was incubated overnight at 4°C, and washed with TBST for three times, with 5 min for each time. An HRP secondary antibody was diluted in 1:10000, incubated at room temperature for 1 hour, and washed with TBST for three times, with 5 min for each time. Western blot was developed by ECL chemiluminescence. About 20 Drosophila was taken for each test, and the test was repeated for three times, from which similar results were derived.

Tyrosine hydroxylase (TH) is a dopaminergic neuron marker, TH level reduction usually represents the death of dopaminergic neurons. Compared with control Drosophila, the DaGS>SNCA Drosophila had an obviously elevated α-synuclein protein level and an obviously reduced TH protein level. TLK could reduce the alpha-synuclein protein level in the DaGS>SNCA Drosophila and partially recover the TH protein level. It was indicated that TLK RNAi also had a certain protection function for alpha-synuclein over-expression Drosophila.

### III. TLK played a vital role in apoptosis induced by alpha-synuclein over-expression or calcium overload

A Drosophila brain tissue was placed and dissected in an HL3 buffer, stained by 1 µM TMRM and incubated in the dark at room temperature for 10 min. Then, the incubated brain tissue was washed with the HL3 buffer for 3 times, and 10 min for each time.

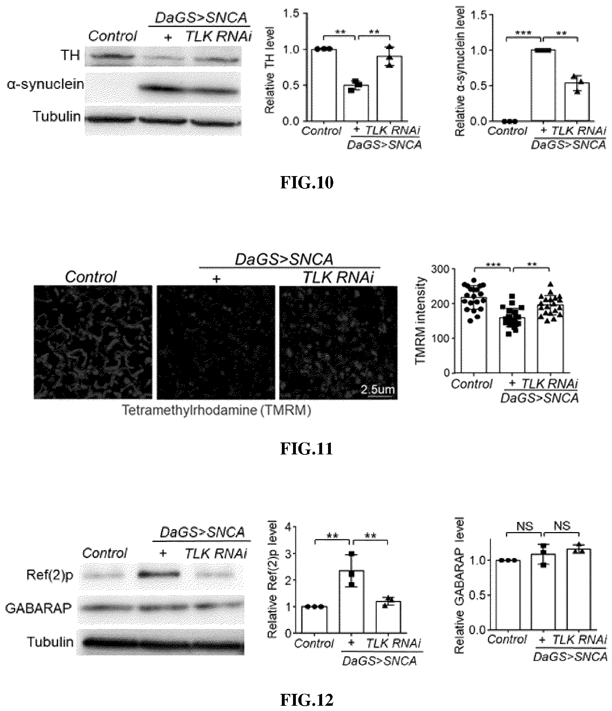

As shown in FIG. 11, red represents tetramethylrhodamin (TMRM); a mitochondrial membrane potential was measured by TMRM, and compared with the control Drosophila, the DaGS>SNCA Drosophila exhibited significantly reduced TMRM fluorescence intensity, but TLK RNAi could obviously recover TMRM fluorescence intensity. It was verified that TLK RNAi could recover the mitochondrial function defect in the DaGS>SNCA Drosophila, indicating that TLK played a vital role in the apoptosis induced by alpha-synuclein over-expression or calcium overload.

### IV. TLK RNAi could remedy autophagy-lysosome dysfunction caused by calcium overload or alpha-synuclein over-expression of Drosophila

The Drosophila flight systemic tissue was collected, and added in an RIPA lysis buffer added with a protease inhibitor and PMSF (50 mM Tris, 150 mM NaCl, 1% Triton X-100, 1% sodium deoxycholate and 0.1% SDS) for lysis on ice for 30 min. Subsequently, ultrasonication was performed on ice under the conditions of 40 w 3 sec on/3 sec off for 1 minute, centrifugation was performed at 12,000 rpm in a centrifugal machine at 4°C for 15 min, a supernatant was collected, diluted by an SDS sample-loading buffer and stewed at 95°C for 5 min. 20-30 µg of a protein sample was loaded, and gel electrophoresis was performed under a constant voltage of 80 V. When bromophenol blue reached the bottom, membrane transfer was performed overnight at a constant current of 130 mA to transfer the protein from a gel onto a PVDF membrane. The membrane was blocked with 5% skim milk at room temperature for 1 hour, and washed with TBST for three times, with 5 min for each time. A primary antibody properly diluted was incubated overnight at 4°C, and washed with TBST for three times, with 5 min for each time. An HRP secondary antibody was diluted in 1:10000, incubated at room temperature for 1 hour, and washed with TBST for three times, with 5 min for each time. Western blot was developed by ECL chemiluminescence.

Compared with Drosophila as a normal control, the DaGS>SNCA Drosophila raised with RU486 had a GABARAP level not obviously changed and an elevated Ref(2)P protein level, but TLK RNAi could reduce the elevated Ref(2)P protein level in the Drosophila with alpha-synuclein over-expression .

### Experimental Test 3 Function of TLK2 Mediation for Calcium-overload Injury

### (I) TLK2 gene knockout could activate autophagy

Hela cells were cultivated in a DMEM medium added with 10% fetal calf serums and 1% streptomycin and penicillin. About fifty thousand cells were seeded in a confocal culture dish, and when a cell density was between 50% and 70%, an LC3-mRFP-GFP adenovirus (HANBIO) was added in 1 mL medium in an amount that was in line with a standard of MOI=10. After transfection for 6-8 hours, the medium was replaced to further cultivate for 24 hours, and then Leica SP8 confocal microscopy was used for the imaging of living cells.

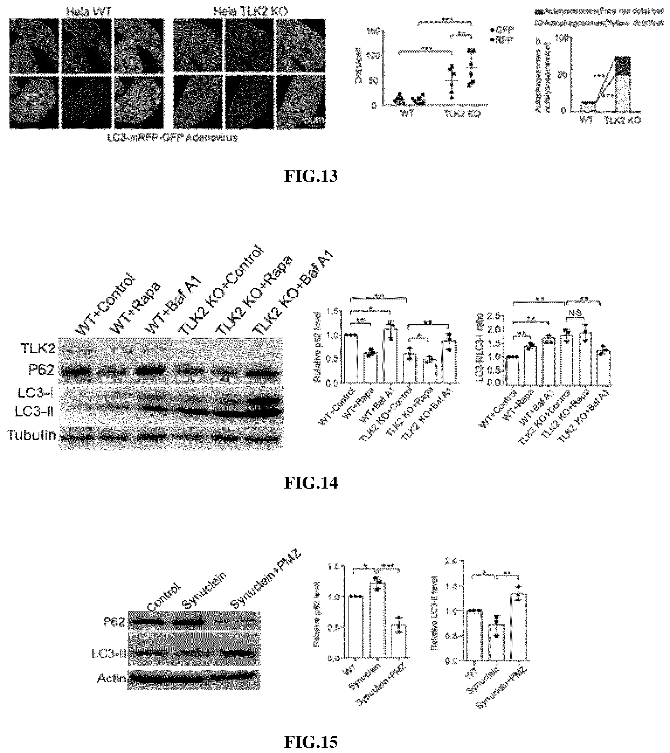

The influence of TLK2 KO on autophagy was tested; a common method for autophagy flux evaluation is to label autophagosomes and autolysosomes by the LC3-mRFP-GFP adenovirus. A low pH value in lysosomes could quench GFP fluorescence signals in the LC3-mRFP-GFP adenovirus; and on the contrary, RFP presented more stable fluorescence signals under a pH value less than 7. In the case of smooth autophagy flux, the number of both yellow and red spots would be increased, respectively representing the increase of the number of autophagosomes and autolysosomes. In FIG. 13, after transfection by the LC3-mRFP-GFP adenovirus for 48 hours, Hela WT and TLK2 KO living cells were imaged. The yellow spots represent autophagosome, and the red spots represent autolysosomes. Compared with wild type cells, TLK2 KO cells had an increased number of yellow and red spots therein, that is, TLK2 KO increased the number of autophagosomes and autolysosomes.

### (II) Analysis of autophagy in TLK 2 KO cells

Hela cells were collected, and were added in an RIPA lysis buffer added with a protease inhibitor and PMSF (50 mM Tris, 150 mM NaCl, 1% Triton X-100, 1% sodium deoxycholate and 0.1% SDS) for lysis on ice for 30 min. Subsequently, ultrasonication was performed on ice under the conditions of 40 w 3 sec on/3 sec off for 1 minute, centrifugation was performed at 12,000 rpm in a centrifugal machine at 4°C for 15 min, a supernatant was collected, diluted by an SDS sample-loading buffer and stewed at 95°C for 5 min. 20-30 µg of a protein sample was loaded, and gel electrophoresis was performed under a constant voltage of 80 V. When bromophenol blue reached the bottom, membrane transfer was performed overnight at a constant current of 130 mA to transfer the protein from a gel onto a PVDF membrane. The membrane was blocked with 5% skim milk at room temperature for 1 hour, and was washed with TBST for three times, with 5 min for each time. A primary antibody properly diluted was incubated overnight at 4°C, and washed with TBST for three times, with 5 min for each time. An HRP secondary antibody was diluted in 1:10000, incubated at room temperature for 1 hour, and washed with TBST for three times, with 5 min for each time. Western blot was developed by ECL chemiluminescence.

In TLK2 KO cells, the LC3-II/LC3-I ratio was increased, and a P62 protein level was reduced (FIG. 14). This indicated that TLK2 KO could activate the autophagy. The autophagy is a dynamic process, for which a level may be elevated accurately through a process of testing an autophagy flux. By using a drug to activate or block an autophagy pathway, the autophagy flux smoothness could be evaluated.

Rapamycin (Rapa) was used for activating the autophagy, and bafilomycin A1 (Baf A1) was used for inhibiting the autophagy. Cells were treated with Rapamycin (Rapa, 40 nM) and bafilomycin A1 (Baf A1, 100nM) for 6 hours, respectively. Western blot results show that in the wild-type Hela cells, Rapa treatment increased the LC3-II/LC3-I ratio and reduced the P62 protein level; and Baf A1 treatment elevated the LC3-II/LC3-I ratio and the P62 protein level. In the TLK2 KO cells, Rapa treatment could further reduce the P62 protein level, and Baf A1 treatment blocked the autophagy activated by TLK2 KO. This indicated smooth autophagy flux in the TLK2 KO cells.

### (III) A TLK2 inhibitor could improve inhibition effect of alpha-syuclein over-expression on the autophagy

SKN cells were collected, and were added in an RIPA lysis buffer added with a protease inhibitor and PMSF (50 mM Tris, 150 mM NaCl, 1% Triton X-100, 1% sodium deoxycholate and 0.1% SDS) for lysis on ice for 30 min. Subsequently, ultrasonication was performed on ice under the conditions of 40 w 3 sec on/3 sec off for 1 minute, centrifugation was performed at 12,000 rpm in a centrifugal machine at 4°C for 15 min, and a supernatant was collected, diluted by an SDS sample-loading buffer and stewed at 95°C for 5 min. 20-30 µg of a protein sample was loaded, and gel electrophoresis was performed under a constant voltage of 80 V. When bromophenol blue reached the bottom, membrane transfer was performed overnight at a constant current of 130 mA to transfer the protein from a gel onto a PVDF membrane. The membrane was blocked with 5% skim milk at room temperature for 1 hour, and washed with TBST for three times, with 5 min for each time. A primary antibody properly diluted was incubated overnight at 4°C, and washed with TBST for three times, with 5 min for each time. An HRP secondary antibody was diluted in 1:10000, incubated at room temperature for 1 hour, and washed with TBST for three times, with 5 min for each time. Western blot was developed by ECL chemiluminescence.

FIG. 15 shows that alpha-synuclein over-expressed lentiviruses in neuroblastoma (SKN-SH) cells; and compared with cells as a normal control, the alpha-synuclein over-expressed cells had a relatively low LC3-II level and a relatively high P62 level, indicating that the alpha-synuclein over-expression reduced the autophagy function. Addition of a known TLK2 kinase inhibitor (Pimozide or PMZ) could up-regulate the LC3-II protein level and down-regulate the P62 protein level.

### (IV) TLK2 kinase inhibitor similar to decreased expression of TLK could remedy an autophagy-lysosome defect caused by alpha-synuclein over-expression

SKN cells were washed with PBS for three times, and incubated in 1 µM LysoTracker in the dark at room temperature for 10 min. Then, a PBS buffer was used for washing 3 times, and 10 min for each time. The Leica SP8 confocal microscopy was used for acquiring images.

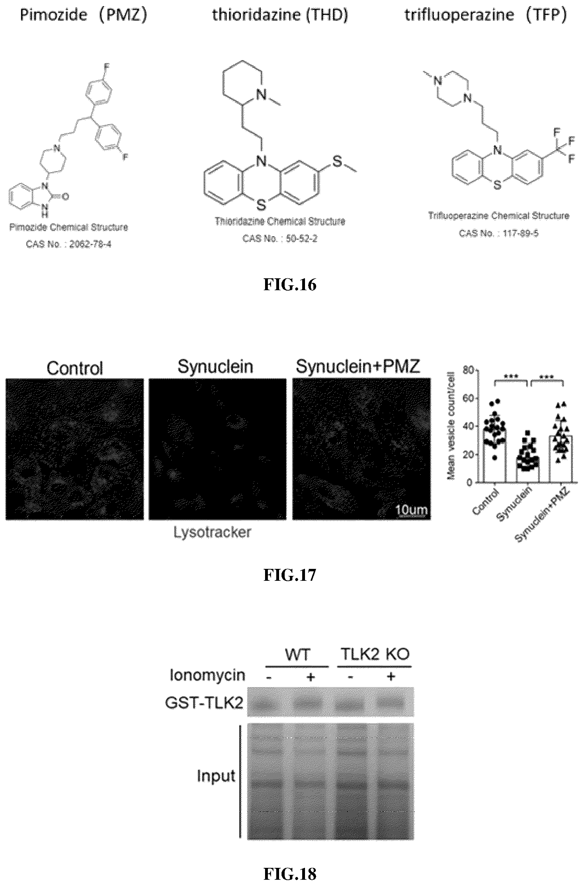

As shown in FIG. 17, red represents LysoTracker signals; the neuroblastoma (SKN-SH) cells had alpha-synuclein over-expressed lentivirus therein for 48 hours, and compared with cells as a normal control, the alpha-synuclein over-expression cells had reduced LysoTracker signals, but PMZ could recover LysoTracker signals. This indicated that the TLK2 kinase inhibitor similar to the decreased expression of TLK could remedy the autophagy-lysosome defect caused by alpha-synuclein over-expression, and the PMZ could recover the autophagy level reduced by the alpha-synuclein over-expression. These results prompted that under the condition of alpha-synuclein over-expression or calcium overload, TLK2 could be probably activated, resulting in autophagy-lysosome function reduction. Therefore, TLK2 knockout or addition of the TLK2 kinase inhibitor could activate the autophagy-lysosome function.

### (V) Calcium overload caused TLK2 hyperphosphorylation and activated kinase activity thereof

TLK2 cDNA was cloned to a PGEX-6P-1 vector containing a GST tag, a recombinant plasmid was expressed into BL21 chemical competent cells for induced expression at 180 rpm at 20°C for 20 hours under an induction condition of 0.5 mM IPTG. GST-TLK2 was purified according to the standard steps of Beyotime GST tag protein purification kit. Hela cells were treated with 17 µM ionomycin for 3 hours, and when a cell density reached 70%, the cells were collected. Lysis was performed in a lysis buffer (10 mM Tris, 150 mM NaCl, 2 mM EDTA and 0.5% TritonX-100). Centrifugation was performed at 12,000 rmp at 4°C for 15 min, and a supernatant was collected. 3 µg of a GST-TLK2 purified protein and 200 µg of a cell lysis buffer were mixed, and placed overnight in a vertical shaker at 4°C. Subsequently, Glutathione Sepharose 4B condensate beads were added, and rotation proceeded at room temperature for 1 hour. The mixture was washed with WB and IP lysis buffers for 6 times, each of which the mixture was centrifugated in a centrifugal machine at 2,000 rmp at 4°C for 5 min. The product was diluted by an SDS sample-loading buffer and stewed at 95°C for 5 min, or a TLK2 protein was cleaved overnight by PreScission Protease at 4°C. The final result was obtained by Commassie staining.

As shown in FIG. 18, to detect whether TLK2 was activated under calcium overload, a GST-TLK2 plasmid was constructed and the TLK2 protein was purified by GST pull down. The purified TLK2 protein was incubated with a cell lysis buffer (inducing calcium overload) treated with ionomycin or a normal cell lysis buffer. By the Commassie staining, it was found that a protein band moved upward after GST-TLK2 was incubated with the cell lysis buffer treated with the ionomycin, indicating that the TLK2 protein was probably modified. Similar change also appeared after the TLK2 protein was incubated with a TLK2 KO cell lysis buffer treated with the ionomycin.

### (VI) After ionomycin treatment, both the normal cell lysis buffer and the TLK2 KO cell lysis buffer could increase TLK2 phosphorylation

TLK2 cDNA was cloned to a PGEX-6P-1 vector containing a GST tag, a recombinant plasmid was expressed into BL21 chemical competent cells for induced expression at 180 rpm at 20°C for 20 hours under an induction condition of 0.5 mM IPTG. GST-TLK2 was purified according to the standard steps of Beyotime GST tag protein purification kit. Hela cells were treated with 17 µM ionomycin for 3 hours, and when a cell density reached 70%, the cells were collected. Lysis was performed in a lysis buffer (10 mM Tris, 150 mM NaCl, 2 mM EDTA and 0.5% TritonX-100). Centrifugation was performed at 12,000 rmp at 4°C for 15 min, and a supernatant was collected. 3 µg of a GST-TLK2 purified protein and 200 µg of a cell lysis buffer were mixed, and placed overnight in a vertical shaker at 4°C. Subsequently, Glutathione Sepharose 4B condensate beads were added, and rotation proceeded at room temperature for 1 hour. The mixture was washed with WB and IP lysis buffers for 6 times, each of which the mixture was centrifugated in a centrifugal machine at 2,000 rmp at 4°C for 5 min. The product was diluted by an SDS sample-loading buffer and stewed at 95°C for 5 min, or a TLK2 protein was cleaved overnight by PreScission Protease at 4°C. The final result was obtained by phos-tag SDS-PAGE.

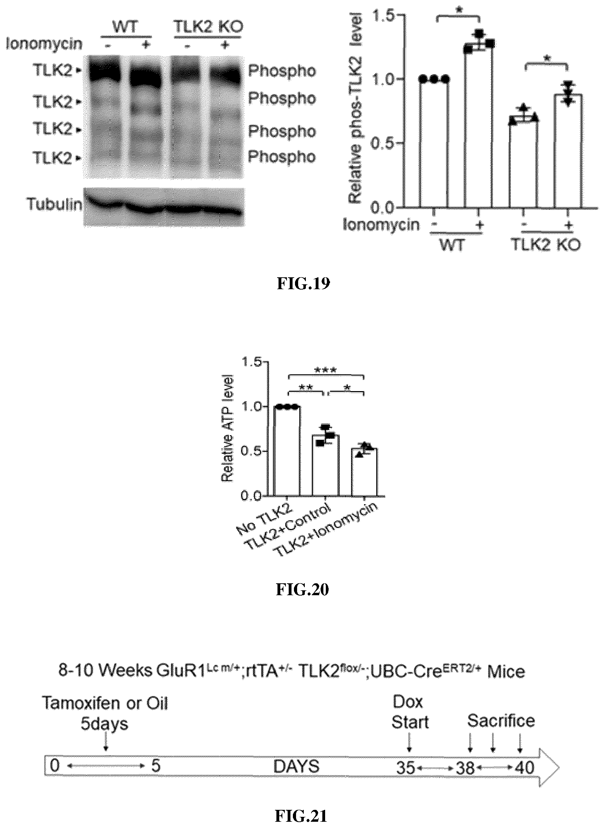

As shown in FIG. 19, a phos-tag SDS-PAGE method was used to further detect whether the upward movement of the GST-TLK2 protein band was modified by phosphorylation. Phos-tag was a functional molecule which could be in specific binding with phosphate ions. It could be used for separating phosphorylated proteins. In normal Western blot, Mn²⁺ and Phos-tag were added, and a non-phosphorylated protein antibody could be used for testing. In Phos-tag SDS-PAGE, a non-phosphorylated protein had a high migration speed, and a phosphorylated protein had a low migration speed. The content of the phosphorylated protein could be analyzed by the change in the migration rate. The purified TLK2 protein was incubated with a cell lysis buffer (inducing calcium overload) treated with the ionomycin or a normal cell lysis buffer. The results showed that after ionomycin treatment, both the normal cell lysis buffer and the TLK2 KO cell lysis buffer could promote TLK2 phosphorylation.

### (VII) Calcium overload could promote TLK2 phosphorylation

TLK2 cDNA was cloned to a PGEX-6P-1 vector containing a GST tag, a recombinant plasmid was expressed into BL21 chemical competent cells for induced expression at 180 rpm at 20°C for 20 hours under an induction condition of 0.5 mM IPTG. GST-TLK2 was purified according to the standard steps of Beyotime GST tag protein purification kit. Hela cells were treated with 17 µM ionomycin for 3 hours, and when a cell density reached 70%, the cells were collected. Lysis was performed in a lysis buffer (10 mM Tris, 150 mM NaCl, 2 mM EDTA and 0.5% TritonX-100). Centrifugation was performed at 12,000 rmp at 4°C for 15 min, and a supernatant was collected. 3 µg of a GST-TLK2 purified protein and 200 µg of a cell lysis buffer were mixed, and placed overnight in a vertical shaker at 4°C. Subsequently, Glutathione Sepharose 4B condensate beads were added, and rotation proceeded at room temperature for 1 hour. The mixture was washed with WB and IP lysis buffers for 6 times, each of which the mixture was centrifugated in a centrifugal machine at 2,000 rmp at 4°C for 5 min. The product was diluted by an SDS sample-loading buffer and stewed at 95°C for 5 min, or a TLK2 protein was cleaved overnight by PreScission Protease at 4°C. The final result was obtained by mass spectrometry.

All serine/threonine phosphorylation sites of TLK2 were identified by mass spectrometry. Under normal circumstances (not treated with the ionomycin), 12 sites were phosphorylated, wherein a control group and an ionomycin treatment group totally had 10 same phosphorylation sites (S102, S114, S116, S133, T160, S222, S329, S375, S392 and S449), and the control group had 2 unique phosphorylation sites (S225 and S376). Compared with the control group, the ionomycin treatment group had 15 additional phosphorylation sites (S25, S72, S109, S110, T207, S209, T212, S217, S277, T299, T300, T366, S616, S751 and S752), but was lacking in 2 phosphorylation sites (S225 and S376) (see Table 3), indicating that the calcium overload could promote TLK2 phosphorylation.

**Table 3 TLK2 Phosphorylation Sites**

| **Sequence** | **Control** + **Ionomycin** | **Sequence** | **Ionomycin unique** |
|---|---|---|---|
| SVPPVAR | S102 | FTGVGVSK | S25 |
| SSPQHSLSNPLPR | S114 | KAEPYETSOG,KGTPR | S72 |
| SSPQHSLSNPLPR | S116 | SSPQHSLSNPLPR | S109 |
| RVEQPLYGLDGSAAK | S1313 | SSPQHSLSNPLPR | S110 |
| LDTEQLAQR | T160 | QTQSDLTIEK | T207 |
| ISALENSKNSDLEKK | S222 | QTQSDLTIEK | S209 |
| INSQREEIER | S329 | QTQSDLTIEK | T212 |
| TNGAENETPSSGNTELK | 5375, | ISALENSK | S217 |
| DTAPALGAHSLLR | S392 | LLIEKSKQEK | S277 |
| IHNEDNSQFKDHPTLNDR | S449 | LGHFTTVR | 1'299 |
| | | LGHFTTVR | T300 |
| **Sequence** | **Control unique** | SKTNGAENETPSSGNTELK | T366 |
| ISALENSKNSDLEKK | S225 | ITDFGLSK | S616 |
| TNGAENETPSSGNTELK | S376 | KSVSTSSPAGAA | S751 |
| | | KSVSTSSPAGAA | ST52 |

### (VIII) Calcium overload could activate TLK2 by promoting TLK2 phosphorylation

1 µL of ATP (0.2 mM), 1 µg of a TLK2 purified protein and 20 µg of a substrate were added to a 50 µL reaction system, the remaining volume was supplemented with a kinase buffer (10 mM Tris pH 7.5, 50 mM KCL, 10 mM MgCl2 and 1 mM DTT), and a reaction was performed at room temperature for 15 min. An MBP protein was taken as a positive control substrate, and a PGK1 protein was taken as a negative control substrate. 50 ul of a Kinase-Lumi^{™} superstrong chemiluminescence kinase assay reagent was added and uniformly mixed to have a reaction in the dark at room temperature for 10 min. Then, a multifunctional microplate reader was used for chemiluminescence testing.

TLK2 kinase activity was verified by an in-vitro kinase experiment. In the in-vitro kinase experiment, a myelin basic protein (MBP) could be used as a substrate for multiple kinases, a group to which a TLK2 (No TLK2) protein was not added was taken as a control, and in a normal treatment (TLK2+Control) and an ionomycin treatment group (TLK2+Ionomycin), the same amount of the TLK2 protein reacted with the same amount of the MBP protein. A reduced ATP level reflected the TLK2 kinase activity. Compared with a normal control group, both the normal treatment and the ionomycin treatment group would consume ATP. Compared with the normal treatment group, the ionomycin treatment group could consume much ATP. This indicated that the calcium overload could improve the TLK2 kinase activity. The above results showed that the calcium overload could activate TLK2 by promoting TLK2 phosphorylation.

### Experimental Test 4

### I. Construction of TLK2 gene knockout could remedy mice under a calcium-overload model

An Lurcher mutant (GluR1Lc) of a glutamate receptor 1 could form constitutive type open cation channels. Transgenic mouse conditionally expressing GluRlLc (GluR1Lc m/m, "m" represents GluR1Lc) were constructed. After hybridization with a doxycycline (dox) induced promoter (rtTA), GluRlLc m/+ appeared; and when doxycycline was provided, rtTA+/- mice could generally express GluRlLc in the whole body. It was found that provision of doxycycline with a normal concentration (2 mg/mL) in drinking water for the mice could induce death in the mice within one day. After doxycycline was diluted by 200 folds (0.01 mg/mL), the GluRlLc mice could survive for more than 3 days. Therefore, the 0.01 mg/mL doxycycline was provided in the drinking water for the mice to slowly induce GluRlLc expression. In addition, conditional TLK2 knockout mice (TLK2flox/flox; UBC-CreERT2/+) were further constructed. The transgenic mice conditionally expressing GluRlLc were hybridized with the conditional TLK2 knockout mice to obtain the required genotypes (GluR1Lc m/+; rtTA + / -; TLK2flox / -; UBC-CreERT2/+).

All mice were raised in the Animal Center of Capital Medical University. The animal experiment was approved by the Animal Ethics Committee of Capital Medical University (Approval No.: AEEI-2015-156). The GluRlLc mice were purchased from Shanghai Model Organisms Center, Inc. In the project, according to the homologous recombination principle, an SA-polyA-Insulator-Insulator-TRE-miniCMVpromoter-Grial (Mut)-wpre-pA- Insulator- Insulator-FRT -PGK-Neo-pA-FRT expression cassette was inserted at a directed Rosa26 (ENSMUSG00000086429) gene site by using an ES cell targeting method.

A brief process is as follows: an ES cell targeting vector was constructed by an In-Fusion cloning method, wherein the vector contained a 3.3 kb 5' homologous arm, an SA-poly A- Insulator- Insulator- TRE-miniCMVpromoter-Grial (Mut)-wpre-pA-Insulator -Insulator-FRT-PGK-Neo-pA-FRT, a 3.2 kb 3' homologous arm and an MC1-TK-polyA and a negative selection marker. After being linearized, the vector was electrically transfected with C57BL/6 ES cells. After G418 and Ganc drug screening, totally 144 resistant ES cell clones were obtained; and by long-range PCR identification, totally 9 correct homologous-recombination positive clones were obtained. An amplified positive ES cell clone was injected into a blastocyst of a C57BL/6J mouse to obtain a chimeric mouse. A highly-proportioned chimeric mouse mated with an flp mouse to obtain 3 positive-flp-gene and Neo-removal mice of positive F1 generation. The GluRlLc m/m (ENSG00000155511) transgenic mouse was hybridized with an rtTA+/+ mouse, and when the mouse grew to about 8-10 weeks, doxycycline was added to the drinking water to induce GluRlLc expression.

The test was designed as shown in FIG. 21, tamoxifen (with corn oil as a control) was continuously and intraperitoneally injected into the mice, aged 8-10 weeks, for 5 days to induce TLK2 knockout. After 1 month, doxycycline (0.01 mg/mL) was added in the drinking water to induce GluRlLc expression and thus induce calcium overload.

The TLK2 gene knockout mice were purchased from Shanghai Model Organisms Center, Inc. TLK2 gene (ENSG00000146872) deletion was achieved by a Cre-lox system. According to the homologous recombination principle, flox modification was performed on a TLK2 gene by using a homologous recombination method of fertilized eggs. A brief process is as follows: Cas9 mRNA and gRNA were obtained by an in-vitro transcription method; a homologous recombination vector(donor vector) was constructed by an In-Fusion cloning method, wherein the vector contained a 2 kb 5' homologous arm, a 0.7 kb flox region and a 2.8 kb 3' homologous arm. Cas9 mRNA, gRNA and donor vector were micro-injected into the fertilized eggs of C57BL/6J mice to obtain mice of F0 generation. By long-range PCR identification, totally 1 correct homologous-recombination mouse of F0 generation was obtained; and the mouse of F0 generation was mated with the C57BL/6J mouse to obtain 4 positive mice of F1 generation. The target exon-4 CRISPR-Cas9 technology was used for TLK2flox/flox mice. After the mice mated with UBC-CreERT2/+ mice, and when the mice grew to about 8-10 weeks, tamoxifen (75 mg/kg) was intraperitoneally injected once a day for 5 continuous days, so as to cause TLK2 gene knockout of the systemic tissue.

As shown in FIG. 21, for GluR1Lcm/+, rtTA+/-, TLK2flox/- and UBC-CreERT2/+ mice, aged 8-10 weeks, the tamoxifen (with corn oil as a control) was continuously and intraperitoneally injected for 5 days to induce TLK2 knockout. After 1 month, doxycycline (0.01 mg/mL) was added in the drinking water to induce GluRlLc expression till the death of the mice.

It was found that the mice without TLK2 KO and the mice only expressing GluRlLc started to die on the 3rd day after doxycycline treatment, but the mice with heterozygote TLK2 KO and the GluRlLc mice survived for a longer time (FIG. 21), indicating that TLK2 KO could prolong the life of the calcium-overload mice.

### II. TLK2 KO could prolong the life of the calcium-overload mice

The mice were anesthetized with pentobarbital sodium (80 mg/kg), and subsequently the abdomens and chests of the mice were dissected, an injector was inserted into the ventriculus sinister, the auricula dextra was cleaved, rapid perfusion was performed with 0.9% normal saline till the liver completely became white, and slow perfusion was performed with 4% paraformaldehyde (PFA) to fix tissue. The brain tissue was taken out and placed overnight in 4% PFA, and then gradient dehydration was performed with 10%, 20% and 30% cane sugars. Subsequently, the brain tissue was embedded with OCT and frozen at minus eighty degrees Celsius in a refrigerator. The brain tissue was cut into 10 µm thick coronary slices by a freezing microtome. The brain tissue slices were fixed in 4% PFA at room temperature for 15 min and subsequently perforated with 0.2% PBST (PBS comprising 0.2% Triton X-100) for three times, with 5 min for each time; and then, the brain tissue slices were blocked in 5% BAS at room temperature for 30 min. A primary antibody was diluted to an appropriate concentration by a blocking buffer and incubated overnight at 4°C. The primary antibody was washed with PBS for three times, and 5 min for each time. A secondary antibody was diluted in 1:200, incubated in the dark at room temperature for 1 hour, and washed with PBS for three times, with 5 min for each time. The secondary antibody was stained with a DAPI diluent for 5 min, and washed with PBS for three times, with 5 min for each time. The Leica SP8 confocal microscopy was used for acquiring images.

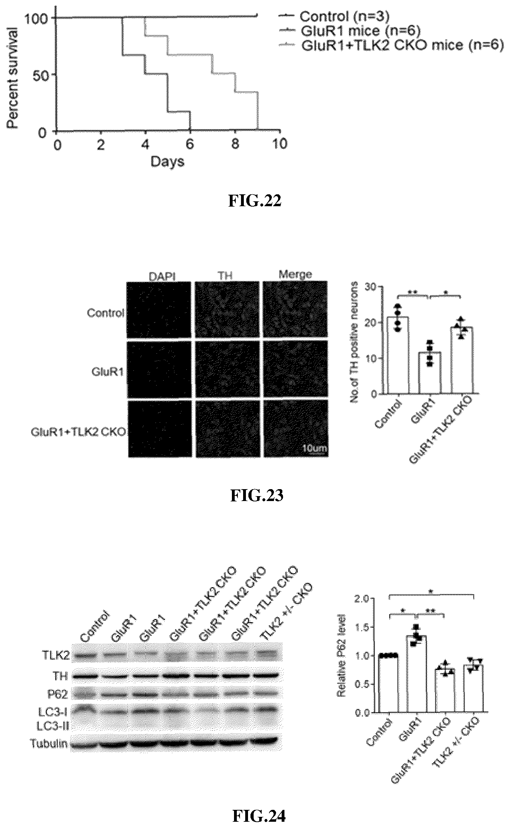

As shown in FIG. 22, the control mice not induced by doxycycline did not die; the GluR1 mice induced only by doxycycline but not by tamoxifen started to die on the third day and could live up to 6 days; the GluR1+ TLK2 CKO mice induced by both doxycycline and tamoxifen started to die on the fourth day and could live up to 9 days.

As shown in FIG. 23, red represents TH positive neurons, and blue represents cell nuclei (DAPI). The immunofluorescent staining results showed that GluR1 mice had a reduced number of TH positive neurons, but TLK2 CKO could partially recover the number of TH positive neurons.

### III. Protection function of TLK2 KO in a calcium-overload mouse model

The moue brain tissue was taken and homogenized in an RIPA lysis buffer added with a protease inhibitor and PMSF (50 mM Tris, 150 mM NaCl, 1% Triton X-100, 1% sodium deoxycholate and 0.1% SDS), and lysis was performed on ice for 30 min. Subsequently, ultrasonication was performed on ice for 1 minute under the conditions of 40 w 3 sec on/3 sec off, centrifugation was performed in a centrifugal machine at 12,000 rpm at 4°C for 15 min, and a supernatant was collected, diluted by an SDS sample-loading buffer and stewed at 95°C for 5 min. 20-30 µg of a protein sample was loaded, and gel electrophoresis was performed under a constant voltage of 80 V. When bromophenol blue reached the bottom, membrane transfer was performed overnight at a constant current of 130 mA to transfer the protein from a gel onto a PVDF membrane. The membrane was blocked with 5% skim milk at room temperature for 1 hour, and washed with TBST for three times, with 5 min for each time. A primary antibody properly diluted was incubated overnight at 4°C, and washed with TBST for three times, with 5 min for each time. An HRP secondary antibody was diluted in 1:10000, incubated at room temperature for 1 hour, and washed with TBST for three times, with 5 min for each time. Western blot was developed by ECL chemiluminescence.

As shown in FIG. 24, Western blot results showed that the calcium-overload mice had a reduced number of both TH protein level and TH positive neurons. But TLK2 KO could partially recover the TH protein level and the number of TH positive neurons. Compared with the calcium-overload Drosophila, the GluRlLc mice had an elevated P62 protein level, without obvious change in LC3-II/I ratio, but TLK2 KO could reduce an elevated P62 protein level.

### IV. TLK2 gene knockout could remedy the mice under an alpha-synuclein over-expression model

C57BL/6 male mice, aged about 8-10 weeks, were taken, anesthetized with pentobarbital sodium (80 mg/kg) and symmetrically fixed on a mouse stereotaxic apparatus. An AAV9-CMV-h-α-synuclein (HANBIO) virus was injected to a right substantia nigra part (AP: -3.0 mm; ML: -1.3 mm; DV: -4.5 mm). 1 µL of the AAV9-CMV-h-α-synuclein (1.5 x 1012 vg/mL) virus or PBS was injected by a 10 µL Hamilton injector at a speed of 0.1 µL /min. After injection, the needle was remained for 5 min and then slowly spun out. After the operation, the mouse was placed on an electric blanket for recovery.

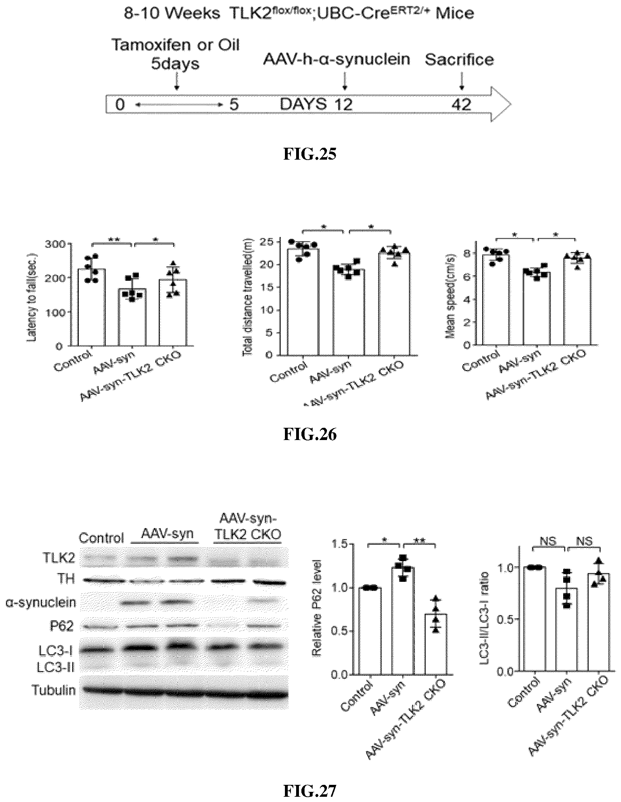

As shown in FIG. 25, homozygous TLK2 KO mice, aged8-10 weeks, (TLK2flox/flox; UBC-CreERT2/+) were intraperitoneally injected with tamoxifen (with corn oil as a control) for 5 consecutive days; after 1 week, the AAV9-CMV-human-alpha-synuclein virus or PBS was injected at the right substantia nigra compacta (SNc) of the mice; and after one month, the mice underwent behavioral testing and were sacrificed.

### V. TLK2 KO mice could recover behavioral abnormality of alpha-synuclein over-expressed mice

In an open field test, the mice adapted to a laboratory environment in a rearing cage for about 30 min, and then the mice were placed in a 50 cm x 50 cm x 50 cm field. After adaption to the environment for 1 min, the mice continuously had activity in the open field for 5 min. After free activity for 5 min was recorded by SMART 3.0 software (RWD, China), the mice were taken back to the rearing cage, and the field was cleaned with 70% ethyl alcohol.

In a rotarod test, the mice adapted to the laboratory environment in the rearing cage for about 30 min and subsequently placed on an accelerated rotating rod with a rotation speed of 5-45 rmp, and the longest duration was 5 min. 2 tests were daily carried out for 3 consecutive days, time for which the mice fell off from a rotarod was recorded, and athletic ability analysis was performed for the last 2 tests on the 3rd day.

As shown in FIG. 26, the left figure is rotarod test results, and the middle and right figures are open-field experimental results. In the rotarod test, the time when the mice fell off from the rotarod was compared among control (a group injected with PBS), AAV-syn (a group injected with alpha-synuclein viruses) and AAV-syn-TLK2 CKO (a group injected with alpha-synuclein viruses in TLK2 CKO mice) mice. In the open-field experiment, the total distance and the average speed of the free activity for 5 min were compared among control (a group injected with PBS), AAV-syn (a group injected with alpha-synuclein viruses) and AAV-syn-TLK2 CKO (a group injected with alpha-synuclein viruses in TLK2 CKO mice) mice. Compared with the control group injected with PBS, alpha-synuclein over-expressed mice had poor behavioral performance. But TLK2 KO mice could recover behavioral abnormality of alpha-synuclein over-expressed mouse.

### VI. TLK2 KO could alleviate these defects

The moue brain tissue was taken and homogenized in an RIPA lysis buffer added with a protease inhibitor and PMSF (50 mM Tris, 150 mM NaCl, 1% Triton X-100, 1% sodium deoxycholate and 0.1% SDS), and lysis was performed on ice for 30 min. Subsequently, ultrasonication was performed on ice for 1 minute under the conditions of 40 w 3 sec on/3 sec off, centrifugation was performed in a centrifugal machine at 12,000 rpm at 4°C for 15 min, and a supernatant was collected, diluted by an SDS sample-loading buffer and stewed at 95°C for 5 min. 20-30 µg of a protein sample was loaded, and gel electrophoresis was performed under a constant voltage of 80 V. When bromophenol blue reached the bottom, membrane transfer was performed overnight at a constant current of 130 mA to transfer the protein from a gel onto a PVDF membrane. The membrane was blocked with 5% skim milk at room temperature for 1 hour, and washed with TBST for three times, with 5 min for each time. A primary antibody properly diluted was incubated overnight at 4°C, and washed with TBST three times, with 5 min for each time. An HRP secondary antibody was diluted in 1:10000, incubated at room temperature for 1 hour, and washed with TBST for three times, with 5 min for each time. Western blot was developed by ECL chemiluminescence.

As shown in FIG. 27, Western blot results showed that compared with the left brain (a control group), the right brain had a reduced TH protein level and an elevated alpha-synuclein protein level, but TLK2 KO could recover the reduced TH protein level of the alpha-synuclein over-expressed mice and reduce the elevated alpha-synuclein protein level. In addition, the alpha-synuclein over-expressed mice also had an elevated P62 protein level, without obvious change in LC3-II/I ratio, but TLK2 KO could reduce the elevated P62 protein level, and TLK2 gene knockout could improve autophagy reduction caused by alpha-synuclein over-expression.

### VII. TLK2 KO could remedy the number of TH positive neurons, reduced by alpha-synuclein over-expression

Mice were anesthetized with pentobarbital sodium (80 mg/kg), and subsequently the abdomen and chest of the mice were dissected, an injector was inserted into the ventriculus sinister, the auricula dextra was cleaved, rapid perfusion was performed with 0.9% normal saline till the liver completely became white, and slow perfusion was performed with 4% paraformaldehyde (PFA) to fix tissues. The brain tissue was taken out and placed overnight in 4% PFA, and then gradient dehydration was performed with 10%, 20% and 30% cane sugars. Subsequently, the brain tissue was embedded with OCT and frozen at minus eighty degrees Celsius in a refrigerator. The brain tissue was cut into 10 µm thick coronary slices by a freezing microtome.

Brain tissue slices were fixed in 4% PFA at room temperature for 15 min and subsequently perforated with 0.2% PBST (PBS containing 0.2% Triton X-100) for three times, with 5 min for each time; and then, the brain tissue slices were blocked in 5% BAS at room temperature for 30 min. A primary antibody was diluted to an appropriate concentration by a blocking solution and incubated overnight at 4°C. The primary antibody was washed with PBS for three times, and 5 min for each time. A secondary antibody was diluted in 1:200, incubated in the dark at room temperature for 1 hour, and washed with PBS for three times, with 5 min for each time. The secondary antibody was stained with a DAPI diluent for 5 min, and washed with PBS for three times, with 5 min for each time. The Leica SP8 confocal microscopy was used for acquiring images.

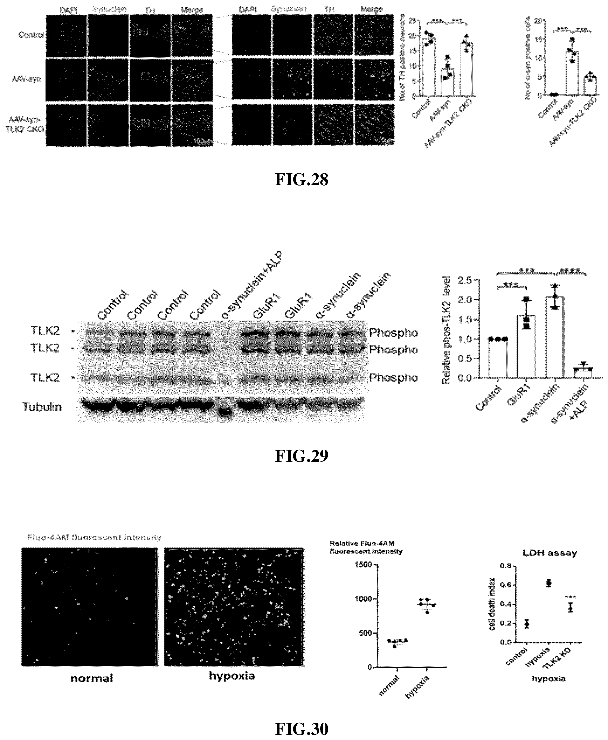

As shown in FIG. 28, red represents TH positive neurons, green represents alpha-synuclein, and blue represents cell nucleus (DAPI). The left white dotted box is further amplified to obtain the right image. The coronary-slice immunofluorescent staining of the mouse brain showed that the lesion side had increased alpha-synuclein aggregation and a reduced number of TH positive neurons, but TLK2 KO could remedy the number of TH positive neurons, reduced by alpha-synuclein over-expression.

### VIII. TLK2 activation under these pathological conditions

The mouse brain tissue was rapidly homogenized in an RIPA lysis buffer added with a protease inhibitor, PMSF and a phosphatase inhibitor, and lysis was performed on ice for 30 min. Centrifugation was performed at 12,000 rmp at 4°C for 15 min, a supernatant was collected, and a part of the supernatant was treated with alkaline phosphatase, diluted with an SDS sample-loading buffer and stewed at 95°C for 5 min. 40 µmol/L MnCh and 20 µmol/L phos-tag were added to 8% SDS-PAGE gel. After electrophoresis, a transfer buffer containing 5 mM EDTA was used for cleaning the gel for 2 times so as to remove divalent cations, with 20 min for each time. Then, the gel was washed with a normal transfer solution for 10 min. Then, a protein was transferred from the gel to a PVDF membrane. The remaining steps were the same as a normal Western blot process.

As shown in FIG. 29, under the pathological conditions of alpha-synuclein over-expression and calcium overload, hyperphosphorylation occurred at TLK2. TLK2 phosphorylation was tested by a phos-tag SDS-PAGE method. Alkaline phosphatase (ALP) was used for dephosphorylation of serine, threonine and tyrosine residues. After testing, increased TLK2 phosphorylation was found in the brains of the GluRlLc and alpha-synuclein over-expressed mice. This indicated that TLK2 was activated under these pathological conditions.

### IX. TLK2 gene knockout could remedy a cell model and an animal model for glaucoma

RGC-5 cells were washed with PBS for 3 times, and incubated in 5 µM Fluo4-AM and 0.02% Pluronic F-127 in the dark at room temperature for 30 min. Then, PBS was used for washing 3 times, with 10 min for each time. The Leica SP8 confocal microscopy was used for imaging and fluorescence intensity measurement.

The calcium-overload occurred at multiple cell types related to the glaucoma, because intraocular hypertension would cause blood flow blocking and cell hypoxia. The cell hypoxia would reduce mitochondrial function, thereby failing to maintain the cell ATP level and causing the calcium overload.

As shown in FIG. 30, the calcium overload occurred at human-derived retina epidermic cells under a low oxygen condition. After raising with 1% oxygen for 24 hours, a relative intracellular calcium ion concentration could be indicated against Fluo-4AM fluorescence intensity. Low oxygen caused the calcium overload. In the TLK2 gene knockout cells, the apoptosis (LDH assay) induced by the low oxygen could be inhibited.

An appropriate number of cells were seeded to a 96-well cell culture plate according to the cell size and the growth speed, so that the cell density during testing did not exceed 80-90%. The culture solution was removed, and the cells were washed with PBS. A fresh culture solution was used (it was recommended to use a low serum culture solution containing 1% serum or a suitable serum-free culture solution), and various culture wells were divided into the following groups: cell-free culture solution wells (background blank control wells), control cell wells not treated with a drug (sample control wells), cell wells used for subsequent lysis, not treated with a drug (maximum sample enzyme activity control wells), and drug-treated cell wells (drug-treated sample wells), and these wells were labeled properly.

Proper drug treatment was provided according to experiment requirements, for example, about 0-10 µL of a specific drug was added for stimulation; different concentrations and different treatment time could be set, and suitable drug solvent was required to be added to the control wells for continued culture as routine against the control. At 1 hour before a predetermined testing time point, the cell culture plate was taken out of a cell incubator, an LDH release reagent provided by a reagent kit was added to the "maximum sample enzyme activity control wells", and the addition amount was equal to 10% of the volume of the original culture solution. After the addition of the LDH release reagent, the cells were blown repeatedly for several times for uniform mixing, and then were incubated in the cell incubator.

After the predetermined time expired, the cell culture plate was centrifuged with a multi-well plate centrifugal machine at 400 g for 5 min. 120 µL of a supernatant was respectively taken from each well and added to corresponding wells of a new 96-well plate for subsequent sample testing. 60 µL of an LDH test working solution was respectively added to each well. After uniform mixing, incubation was performed in the dark at room temperature (about 25°C) for 30 min (the plate could be wrapped with an aluminum foil and then placed for slow shaking in a horizontal shaking table or a side-sway shaking table). Then, absorbance was measured at 490 nm. Any wavelength of 600 nm or greater than 600 nm was used as a reference wavelength for dual-wavelength determination. Calculation was performed (the background blank control well absorbance was deducted from the measured absorbance of each group).

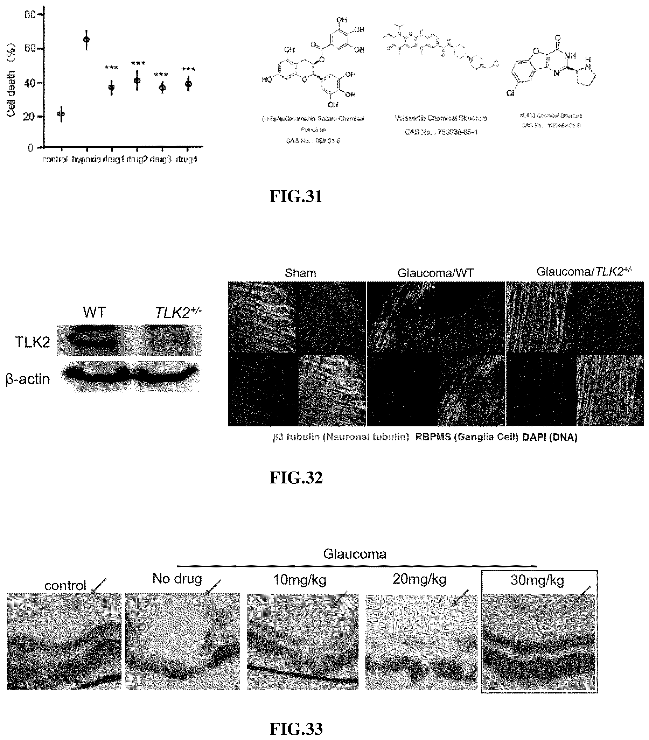

As shown in FIG. 31, it was found that a new micro-molecule had calcium-overload injury inhibition activity. A cell calcium-overload model was used for drug screening in a micro-molecule library (over 2,000 active micro-molecules), and drug1 (PMZ) was taken as a positive control. Drug 2 to Drug 4 respectively represented Volasertib, Epigallocatechin Gallate and XL413.

Normal saline was injected into a vitreous body to establish an acute intraocular-hypertension glaucoma model, and the intraocular pressure of the mouse was tested by an intraocular pressure instrument, which was at 50-70 mmHg. After 48 hours, retina was obtained. Immunohistochemical staining was performed; RGC cell nucleus, RGC neural tree and cell death marker were respectively labeled. Compared with wild type mice of the same generation, apoptosis was found.

As shown in FIG. 32, mouse TLK2 gene knockout had an effect in glaucoma injury antagonism. WB diagram shows that TLK2 heterozygote knockout really reduced the TLK2 protein level. βbeta-actin labeled the protein loading amount. The right images are mouse retina fluorescence immunoassay results. Sham is an untreated normal mouse retina. A beta 3 tubulin antibody was stained to label a tubulin protein in neuron (green), an RBPMS antibody was stained to label optic-nerve soma (red), and DAPI was stained to label DNA (i.e., cell nucleus, blue). The glaucoma model is an acute intraocular hypertension model. The results in the figure showed that the glaucoma model caused neurotubule staining reduction and breakage as well as a reduced number of retinal ganglion cells. In the context of TLK2 gene knockout, the above injuries were significantly reduced.

Normal saline was injected into a vitreous body to establish an acute intraocular-hypertension glaucoma model, and the intraocular pressure of the mouse was tested by an intraocular pressure instrument, which was at 50-70 mmHg. After 6 hours, a drug was injected intraperitoneally. After 48 hours, retina was obtained. Or after an AAV2 virus was injected into fundus oculi to express TLK2 sh RNA for two weeks, intraocular hypertension treatment was performed; and after 48 hours, the retina was taken.

As shown in FIG. 33, PMZ had an effect in glaucoma injury antagonism. Neurons in the retina were labeled by Nissl staining. Arrows indicate retinal ganglion layers. Control is an untreated normal mouse retina. No drug refers to failure in drug treatment after glaucoma modeling. 10 mg/kg to 30 mg/kg are the dose for PMZ intraperitoneal injection (once a day). The results in the figure showed that the glaucoma model caused the reduced number of the neurons of the retinal ganglion layer; but high-dose PMZ (30 mg/kg) had a protection function. A similar method validated that Epigallocatechin Gallate and the AAV2 virus had similar efficacy in expressing the sequences (GACCGCTTGAGACTGGGCCACTTTA; CCATTATCATGCAGATTGT; GTCGGATCCTTGAGTGATA) of TLK2 sh RNA.

X. TLK2 siRNA and AAV virus expressed shRNA such as artificially synthesized nucleotide sequences 1-33 verified TLK2 RNAi knockdown efficiency (FIG. 32).

SKN tissue was collected, and added in an RIPA lysis buffer added with a protease inhibitor and PMSF (50 mM Tris, 150 mM NaCl, 1% Triton X-100, 1% sodium deoxycholate and 0.1% SDS) for lysis on ice for 30 min. Subsequently, ultrasonication was performed on ice for 1 minute under the conditions of 40 w 3 sec on/3 sec off, centrifugation was performed in a centrifugal machine at 12,000 rpm at 4°C for 15 min, and a supernatant was collected, diluted by an SDS sample-loading buffer and stewed at 95°C for 5 min. 20-30 µg of a protein sample was loaded, and gel electrophoresis was performed under a constant voltage of 80 V. When bromophenol blue reached the bottom, membrane transfer was performed overnight at a constant current of 130 mA to transfer the protein from a gel onto a PVDF membrane. The membrane was blocked with 5% skim milk at room temperature for 1 hour, and washed with TBST for three times, with 5 min for each time. A primary antibody properly diluted was incubated overnight at 4°C, and washed with TBST three times, with 5 min for each time. An HRP secondary antibody was diluted in 1:10000, incubated at room temperature for 1 hour, and washed with TBST for three times, with 5 min for each time. Western blot was developed by ECL chemiluminescence.

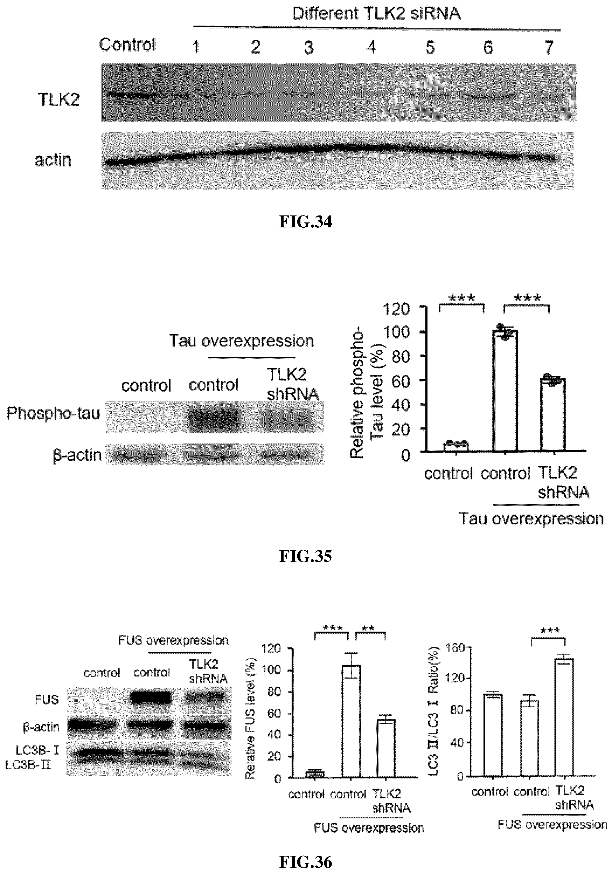

As shown in FIG. 34, multiple human-derived TLK2 RNAi sequences could reduce a TLK2 protein level. Different TLK2 siRNAs were transfected in human-derived SKN cells. 7 sequences listed herein were randomly synthesized from 33 sequences mentioned above, which could reduce the TLK2 protein level in different degrees.

### XI. TLK2 shRNA could reduce tau protein packing related to the Alzheimer's disease

A lentivirus expressed human-derived tau protein was infected in a human-derived neuroma cell line (SKN) to establish a stably expressed cell line. Then, lentivirus was used to infect TLK2 shRNA. After culture for 72 hours, cells were lysed and proteins were collected. Western blots were conducted, and beta-actin was taken as a protein sample-loading control. The experiment was repeated for three times.

As shown in FIG. 35, TLK2 (shRNA) knockdown could reduce an intracellular tau protein level, tau was over-expressed in the human-derived neuroma cell line (SKN), and TLK2 shRNA was infected with lentivirus.

### XII. TLK2 shRNA could reduce FUS protein packing related to the amyotrophic lateral sclerosis

Lentivirus infection was performed in mouse primary-culture neurons to express FUS; and this protein is a human mutant pathogenic protein FUS (P525L). After a stably expressed FUS (P525L) cell line was established, the lentivirus infection was performed to express TLK2 shRNA. After culture for 72 hours, cells were lysed and proteins were collected. Western blots were conducted, and beta-actin was taken as a protein sample-loading control. A high LC3II/I ratio represents active autophagy. The experiment was repeated for three times.

As shown in FIG. 36, TLK2 (shRNA) knockdown could reduce an intracellular FUS protein level and elevate an autophagy level. FUS was over-expressed in the mouse primary-culture neurons (pathogenic FUS mutant, which caused the amyotrophic lateral sclerosis in a patient). TLK2 shRNA was infected with lentivirus.

Therefore, the present invention employs the marker of the calcium-overload-mediated neuronal death and the application thereof, wherein the calcium overload and TLK2 hyperphosphorylation as new molecular markers for predicting a disease occurrence progress or testing a therapeutic effect not only have a rapid prewarning function for the Alzheimer's disease, the Parkinson's disease, the amyotrophic lateral sclerosis and the glaucoma, but also have a main therapeutic effect in processes of the drugs prepared for treating aforementioned diseases.

Finally, it should be noted that the above embodiments serve only to illustrate the technical solutions of the present invention and not to limit the same; although the present invention has been described in detail with reference to the preferred embodiments, it should be understood by a person of ordinary skill in the art that the technical solutions described in the present invention can still be amended or equivalently replaced; and the amendment or equivalent replacement should not make the amended technical solutions deviate from the spirit and scope of the technical solutions of the present invention.

## Claims

1. An application of a marker of a calcium-overload-mediated neuronal death in preparation of a drug for treating multiple aging-related neurodegenerative diseases and in a disease testing method.

2. The application according to claim 1, **characterized in that** the diseases comprise, but are not limited to, an Alzheimer's disease, a Parkinson's disease, amyotrophic lateral sclerosis or glaucoma.

3. A marker of a calcium-overload-mediated neuronal death, **characterized in that** the marker is hyperphosphorylated TLK2.

4. The marker of the calcium-overload-mediated neuronal death according to claim 3, **characterized in that** it further comprises a TLK2 inhibiting drug, an RNA interference factor of TLK2, a TLK2 knockout factor or a TLK2 knockout RNA interference factor.

5. The marker of the calcium-overload-mediated neuronal death according to claim 4, **characterized in that** the TLK2 inhibiting drug comprises, but is not limited to, pimozide, thioridazine or trifluoperazine.

6. The marker of the calcium-overload-mediated neuronal death according to claim 4, **characterized in that** the RNA interference factor of TLK2 is microRNA interference, antisense RNA or other TLK2 mRNA transcription and protein-translation inhibiting factors.

7. The marker of the calcium-overload-mediated neuronal death according to claim 6, wherein RNA interference sequences of TLK2 are nucleotide sequences as shown in SEQ ID NO. 1-33.
